# EUROPEAN PATENT APPLICATION

(11) **EP 3 246 020 A1**
(43) Date of publication of application: **22.11.2017**
(21) Application number: 17171787.9
(22) Date of filing: 18.05.2017
(51) Int. Cl.: A61K 9/20, A61K 9/48, A61K 31/4439

(54) **NEW ORAL PHARMACEUTICAL FORMULATIONS OF DABIGATRAN**

(30) Priority: 20.05.2016 TR 201606697
(71) Applicant: Sanovel Ilac Sanayi ve Ticaret A.S., 34460 Istanbul (TR)
(72) Inventor: TÜRKYILMAZ, Ali, 34460 Istanbul (TR); YELKEN, Gülay, 34460 Istanbul (TR); GÜLKOK, Yildiz, 34460 Istanbul (TR)
(74) Representative: Sevinç, Erkan

(57) **Abstract**

The present invention relates to new oral pharmaceutical formulations comprising dabigatran etexilate free base and cyclodextrin with least one pharmaceutically acceptable excipient.

## Description

### Field of Invention

The present invention relates to new oral pharmaceutical formulations comprising dabigatran etexilate free base and cyclodextrin with at least one pharmaceutically acceptable excipient.

### The background of the invention

Dabigatran etexilate (Formula I), which is already known from WO 98/37075, is a competitive reversible non-peptide antagonist of thrombin. It is a direct thrombin inhibitor indicated to reduce the risk of stroke and systemic embolism in patients with non-vascular atrial fibrilation. Thrombin is a multifunctional enzyme which converts fibrinogen to fibrin, crosslinking fibrin monomers via activation of factor XIII and augmenting further thrombin production via the activation of factors V and VIII. It also activates platelets, generates anticoagulant activity via activation of protein C and initiates numerous cellular processes.

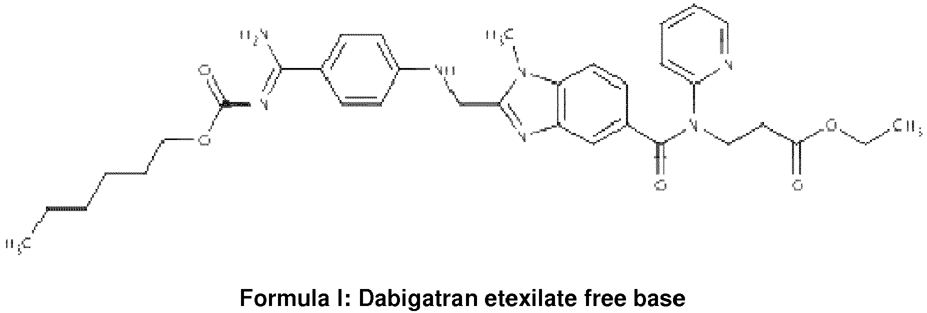

The methane sulphonic acid addition salt of dabigatran etexilate, which is commercially available under the trade name PRADAXA® (in the strength of 75, 110, 150mg), pellet composition of dabigatran etexilate mesylate (DEM) is also disclosed in EP1870100. This composition is formulated with a core material consisting of organic acid and an active layer which covers the core.

Apart from the methanesulfonate salt of dabigatran etexilate, other acid addition salts of the compound are provided in prior art. For example, WO2012/077136 is directed to the oxalate salt of dabigatran etexilate and besides, its hydrochloride salt is identified in EP1877395.

These various dabigatran etexilate salts disclosed in prior art, were compared to their physicochemical properties like water solubility and stability which are important for the development of pharmaceutical formulations. Also, it is known in the prior art that dabigatran etexilate is a weakly basic compound and therefore it has high solubility in acidic media.

Absorption of the active substances generally occurs in the small intestine due to a large surface area and the slow peristaltic movements of the small intestine that has a basic media. However, absorption from the stomach that has an acidic media is negligibly low due to the fast peristaltic movements and the high the surface area of the stomach. Thus the dissolution of the active substances in the small intestine, a basic media, is important. Dabigatran, is weakly basic, should be dissolved in basic environments and the dissolution of dabigatran should be pH independent.

Thus, there is still a need in the art to develop stable and bioavailable pharmaceutical formulations comprising dabigatran etexilate free base with a pH independent release by an easy process. By this need, the formulation is combined with the use of cyclodextrin to overcome the solubility and stability problems of dabigatran etexilate free base in a safe manner disclosed above.

### Detailed description of the invention

In this present invention, the object is to provide new stable pharmaceutical formulations comprising dabigatran etexilate free base and cyclodextrin, with safe and effective dissolution profile and an easy process.

According to this object, the present invention relates to pharmaceutical formulations comprising dabigatran etexilate free base and cyclodextrin, wherein the amount of dabigatran etexilate free base is between 5.0 to 85.0 % by weight of total formulation and the amount of cyclodextrin is between 1.0 to 50.0% by weight of total formulation.

According to one embodiment, the ratios used in this present invention ensure the required effective dose for the treatment and provide stability for dabigatran etexilate free base.

In this present invention, the use of cyclodextrin is very important to obtain safe and effective dissolution profile and stability for dabigatran etexilate free base. Thus, cyclodextrin helps increasing the solution of dabigatran etexilate free base in a safe manner. Because the majority of pharmaceutical active agents do not have sufficient solubility and traditional formulations for insoluble drugs (i.e. dabigatran etexilate free base) involve a combination of organic solvents, surfactants and extreme pH conditions to help its solubility which often cause irritation or other adverse reactions. Cyclodextrin is not irritant and offer distinct advantages such as the stabilization of active compounds, reduction in volatility of drug molecules and masking of malodours and bitter tastes. Furthermore, there is no need to use a sweetener or flavoring agent to mask bitter or irritant taste of dabigatran etexilate free base.

As a result, in this present invention, the use of cyclodextrin in a proper amounts, results in improved bioavailability and increasing the pharmacological effect allowing a reduction in the dose of dabigatran etexilate free base administered.

According to another embodiment, the use of cyclodextrin in this present invention, improves the stability of dabigatran etexilate free base by increasing its resistance to hydrolysis, oxidation, heat, light and metal salts.

According to one embodiment, in this present invention, dabigatran etexilate free base is used as an anticoagulant, for use in the treatment of cardiac arrhythmia and blood clotting and to prevent serious or life-threatening side effects in patients.

According to a further embodiment, the pharmaceutical formulations of this invention further comprising at least one disintegrant.

Suitable disintegrants may comprise but not limited to sodium starch glycolate, cross-linked polyvinyl pyrrolidone (crospovidone), pregelatinized starch, low-substituted hydroxypropyl cellulose, sodium carboxymethyl cellulose, calcium carboxymethyl cellulose, carboxymethyl cellulose, docusate sodium, guar gum, polyacryline potassium, sodium alginate, corn starch, alginic acid, alginates, ion-exchange resins, magnesium aluminium silica, sodium dodecyl sulphate, poloxamer, sodium glycine carbonate or mixtures thereof. Preferably the disintegrants are sodium starch glycolate and cross-linked polyvinyl pyrrolidone (crospovidone).

According to this embodiment, the desired total amount of disintegrants is between 0.5 - 30% by weight of the total formulation. This amount also helps to ensure the proper dissolution of dabigatran etexilate free base.

According to one embodiment of this invention, the pharmaceutical formulations are in the form of solid dosage form which is administrated orally.

According to this embodiment, the pharmaceutical formulations are in the form of tablets, coated or uncoated tablets, capsules, multilayer tablets, buccal tablets, sublingual tablets, tablet in tablets, in-lay tablets, effervescent tablets, immediate release tablets, modified release tablets, orally disintegrating tablet, film-coated tablets, enteric coated tablets, pills, hard or soft gelatin capsules, powders, mini tablets, pellets, coated bead systems, granules, dragees, sachets, films, orally administrable films. Preferably, said pharmaceutical formulations are in the form of tablet or capsule.

According to the challenges mentioned above the selection of the excipients thus essential. According to this embodiment, at least one pharmaceutically acceptable excipient other than disintegrant, is selected from the group comprising fillers, binders, lubricants, glidants, acids, melting components, plasticizers or mixtures thereof.

Suitable fillers may comprise but not limited to mannitol, microcrystalline cellulose, lactose, dibasic calcium phosphate, spray-dried mannitol, dextrose, sucrose, dicalcium phosphate sorbitol, xylitol, inositol, kaolin, inorganic salts, calcium salts, polysaccharides, sodium chloride, dextrates, lactitol, maltodextrin, sucrose-maltodextrin mixture, trehalose, sodium carbonate, sodium bicarbonate, calcium carbonate or mixtures thereof.

Suitable binders may include but not limited to xanthan gum, polyvinylpyrrolidone, polyethylene glycol, polyvinyl alcohol, starch, glucose, glucose syrup, natural gums, sucrose, sodium alginate, hydroxypropyl methyl cellulose, hydroxypropyl cellulose, carboxy methyl cellulose, methyl cellulose, gelatin, carrageenan, guar gum, carbomer, polymethacrylates, methacrylate polymers, collagens, gelatin, agar, alginate, alginic acid, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

Suitable lubricants may comprise but not limited to magnesium stearate, calcium stearate, zinc stearate, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

Suitable glidants may comprise but not limited to talc, aluminium silicate, silicon dioxide, starch or mixtures thereof.

Suitable acids are selected from the group comprising tartaric acid, citric acid, ascorbic acid, fumaric acid, malic acid, nicotinic acid, adipic acid, acetylsalicylic acid or mixtures thereof.

Suitable melting components are selected from the group comprising gelucire (stearyl macrogol glyceride - stearoyl polyoxylglycerides), poloxamer 188 (polyoxyethylene - polyoxypropylene block copolymer), polyethylene glycol, soluplus, cationic methacrylate,

Suitable plasticizers may comprise but not limited to diethyl phthalate, polyethylene glycols of different molecular weights, propylene glycol, glycerin, triethyl citrate, triacetin, dibutyl phthalate, tributhyl citrate, castor oil, dibutyl sebacate, diacetylated monogglycerides or mixtures thereof.

This invention is further defined by reference to the following examples. Although the examples are not intended to limit the scope of the present invention, they should be considered in the light of the description detailed above.

### Example 1: Direct Compression

% 5.0 - 85.0 dabigatran etexilate free base
% 1.0 - 50.0 cyclodextrin
% 5.0 - 90.0 microcrystalline cellulose
% 1.0 - 30.0 lactose
% 0.5 - 10.0 sodium starch glyconate
% 0.1 - 2.0 silicon dioxide
% 0.25 - 2.0 magnesium stearate
by weight of total formulation.

**Production process:** Dabigatran etexilate free base and Cyclodextrin are mixed by geometric dilution method and other excipients are added continuously. Latest magnesium stearate is added and mixed shortly. Finally, the mixture is pressed as tablets or filled into the capsules. Optionally tablets can be coated with opadry ABM (polivinil alcohol) or Kollicoat IR (polyvinyl alcohol/polyethylene glycol graft copolymer).

### Example 2: Dry Granulation

% 5.0 - 85.0 dabigatran etexilate free base
% 1.0 - 50.0 cyclodextrin
% 1.0 - 30.0 crospovidone
% 5.0 - 90.0 dibasic calcium phosphate
% 0.05 - 20.0 xanthan gum
% 0.1 - 2.0 silicon dioxide
% 0.25 - 2.0 magnesium stearate
by weight of total formulation.

**Production process:** Dabigatran etexilate free base, cyclodextrin, dibasic calcium phosphate, crospovidone, xanthan gum and silicon dioxide are mixed. This mixture is mixed with half amount of magnesium stearate and then granulated with roller compactor. The rest of the magnesium stearate is added to these granules and mixed. Finally, the mixture is pressed as tablets or filled into the capsules. Optionally tablets can be coated opadry ABM (polivinil alcohol) or Kollicoat IR (polyvinyl alcohol/polyethylene glycol graft copolymer).

### Example 3: Hot-melt extrusion

% 5.0 - 85.0 dabigatran etexilate free base
% 1.0 - 50.0 cyclodextrin
% 5.0 - 90.0 mannitol
% 1.0 - 30.0 crospovidone
% 0.5 - 50.0 gelucire
% 0.1 - 2.0 Silicon dioxide
% 0.25 - 2.0 Magnesium stearate
by weight of total formulation.

**Production process:** Dabigatran etexilate free base is mixed with gelucire and melted together and passed from extruder, then cooled and sieved. Cyclodextrin, mannitol, crospovidone, silicon dioxide and magnesium stearate is added to obtained granules and mixed for a while.

Finally, the mixture is pressed as tablets or filled into the capsules. Optionally tablets can be coated opadry ABM (polivinil alcohol) or Kollicoat IR (polyvinyl alcohol/polyethylene glycol graft copolymer).

### Example 4 : Wet Granulation

% 5.0 - 85.0 dabigatran etexilate free base
% 1.0 - 50.0 cyclodextrin
% 1.0 - 30.0 crospovidone
% 5.0 - 90.0 dibasic calcium phosphate
% 0.1 - 20.0 sodium alginate
% 0.1 - 2.0 silicon dioxide
% 0.25 - 2.0 magnesium stearate
by weight of total formulation.

**Production process:** Cyclodextrin solution is prepared by a proper solvent or solvent mixture. Dabigatran etexilate free base, dibasic calcium phosphate and half of the crospovidone are mixed and granule with cyclodextrin solution. These granules are sieved and dried. Dry granules are sieved again and mixed with the rest of the excipients. Then, magnesium stearate is added to this mixture and further mixed again for a short time.

Finally, the mixture is pressed as tablets or filled into the capsules. Optionally tablets can be coated opadry ABM (polivinil alcohol) or Kollicoat IR (polyvinyl alcohol/polyethylene glycol graft copolymer).

### Example 5: Pellet coating

% 5.0 - 85.0 dabigatran etexilate free base
% 1.0 - 50.0 cyclodextrin
% 0.1 - 20.0 pullulan
% 5.0 - 60.0 tartaric acid
% 5.0 - 90.0 sugar pellet
% 0.5 - 40.0 polymethyl methacrylate copolymers (independent from pH)
% 1.0 - 40.0 diethyl phthalate
% 1.0 - 30.0 crospovidone
% 0.1 - 2.0 silicon dioxide
% 0.25 - 2.0 magnesium stearate
by weight of total formulation.

**Production process:** A hydoralcoholic/alcoholic solution of dabigatran etexilate free base and cyclodextrin is prepared by adding pullulan. Sugar pellets are coated with this solution. The sugar pellets which were coated with the active ingredient are coated with polymethyl methacrylate derivatives. These pellets first mixed with silicon dioxide and then with magnesium stearate.

Finally, this pellet mixture is pressed as tablets or filled into the capsules. Optionally tablets can be coated opadry ABM (polivinil alcohol) or Kollicoat IR (polyvinyl alcohol/polyethylene glycol graft copolymer).

## Claims

1. Pharmaceutical formulations comprising dabigatran etexilate free base and cyclodextrin.

2. The pharmaceutical formulations according to claim 1, wherein the amount of dabigatran etexilate free base is between 5.0 to 85.0 % by weight of total formulation.

3. The pharmaceutical formulations according to claim 1, wherein the amount of cyclodextrin is between 1.0 to 50.0% by weight of total formulation.

4. The pharmaceutical formulations according to claim 1, further comprising at least one disintegrant.

5. The pharmaceutical formulations according to claim 4, wherein the disintegrants are selected from the group comprising sodium starch glycolate, cross-linked polyvinil pyrrolidone, magnesium aluminium silica, sodium dodecyl sulphate, sodium carboxymethyl cellulose, carboxymethyl calcium, low substituted hydroxypropyl methyl cellulose, poloxamer or mixtures thereof.

6. The pharmaceutical formulations according to claim 5, wherein the disintegrants are preferably sodium starch glycolate or cross-linked polyvinil pyrrolidone or mixtures thereof.

7. The pharmaceutical formulations according to claim 6, wherein the total amount of disintegrants is between 0.5 - 30% by weight of the total formulation.

8. The pharmaceutical formulations according to claim 1 or 4, further comprising at least one pharmaceutically acceptable excipient other than disintegrant which is selected from the group comprising fillers, binders, lubricants, glidants, acids, melting components, plasticizers or mixtures thereof.

9. The pharmaceutical formulations according to any preceding claims, comprising
a. % 5.0 - 85.0 dabigatran etexilate free base
b. % 1.0 - 50.0 cyclodextrin
c. % 5.0 - 90.0 microcrystalline cellulose
d. % 1.0 - 30.0 lactose
e. % 0.5 - 10.0 sodium starch glyconate
f. % 0.1 - 2.0 silicon dioxide
g. % 0.25 - 2.0 magnesium stearate
by weight of total formulation.

10. The pharmaceutical formulations according to any preceding claims, comprising
a. % 5.0 - 85.0 dabigatran etexilate free base
b. % 1.0 - 50.0 cyclodextrin
c. % 1.0 - 30.0 crospovidone
d. % 5.0 - 90.0 dibasic calcium phosphate
e. % 0.05 - 20.0 xanthan gum
f. % 0.1 - 2.0 silicon dioxide
g. % 0.25 - 2.0 magnesium stearate
by weight of total formulation.

11. The pharmaceutical formulations according to any preceding claims, comprising
a. % 5.0 - 85.0 dabigatran etexilate free base
b. % 1.0 - 50.0 cyclodextrin
c. % 5.0 - 90.0 mannitol
d. % 1.0 - 30.0 crospovidone
e. % 0.5 - 50.0 gelucire
f. % 0.1 - 2.0 silicon dioxide
g. % 0.25 - 2.0 magnesium stearate
by weight of total formulation.

12. The pharmaceutical formulations according to any preceding claims, comprising
a. % 5.0 - 85.0 dabigatran etexilate free base
b. % 1.0 - 50.0 cyclodextrin
c. % 1.0 - 30.0 crospovidone
d. % 5.0 - 90.0 dibasic calcium phosphate
e. % 0.1 - 20.0 sodium alginate
f. % 0.1 - 2.0 silicon dioxide
g. % 0.25 - 2.0 magnesium stearate
by weight of total formulation.

13. The pharmaceutical formulations according to any preceding claims, comprising
a. % 5.0 - 85.0 dabigatran etexilate free base
b. % 1.0 - 50.0 cyclodextrin
c. % 0.1 - 20.0 pullulan
d. % 5.0 - 60.0 tartaric acid
e. % 5.0 - 90.0 sugar pellet
f. % 0.5 - 40.0 polymethyl methacrylate copolymers (independent from pH)
g. % 1.0 - 40.0 diethyl phthalate
h. % 1.0 - 30.0 crospovidone
i. % 0.1 - 2.0 silicon dioxide
j. % 0.25 - 2.0 magnesium stearate
by weight of total formulation.

14. The pharmaceutical formulations according to claims 9 to 13, wherein said pharmaceutical formulations are in the formof tablets, coated or uncoated tablets, capsules, multilayer tablets, buccal tablets, sublingual tablets, tablet in tablets, in-lay tablets, effervescent tablets, immediate release tablets, modified release tablets, orally disintegrating tablet, film-coated tablets, enteric coated tablets, pills, hard or soft gelatin capsules, powders, mini tablets, pellets, coated bead systems, granules, dragees, sachets, films, orally administrable films or solids.

15. The pharmaceutical formulations according to claim 14, wherein said pharmaceutical formulations are in the form of tablet or capsule.
